# EUROPEAN PATENT APPLICATION

(11) **EP 2 444 478 A1**
(43) Date of publication of application: **25.04.2012**
(21) Application number: 10013899.9
(22) Date of filing: 22.10.2010
(51) Int. Cl.: C12M 1/12, C12M 3/06

(54) **Tubular body fluid mass exchange system and mass exchange device**

(71) Applicant: Gerlach, Jörg, Dr. med., 10965 Berlin (DE)
(72) Inventor: Bornemann, Reinhard, Dr., 33604 Bielefeld (DE)
(74) Representative: Pfenning, Meinig & Partner GbR

(57) **Abstract**

The present invention is related to a tubular body fluid mass exchange system which in principle is composed of a tube arrangement with up to 1.000 tubes, wherein each tube either is a microporous tube with pores in the range of 0,01 to 0,5 µm or each tube has at least one mass exchange aperture with a diameter between 0,1 to 3 mm; these tubes are arranged in a way that they comprise at least one collective inlet and at least one collective outlet, in a preferred embodiment one collective inlet leading to apertures in the environment and one collective outlet leading from apertures in the environment to a collective outlet. This system enables mass exchange in the abdomen via the serosa surface and thus extracorporealdetoxification, including albumin dialysis, and regulation. Furthermore, present invention is related to a mass exchange device which comprises a bioreactor for growing and exploiting the metabolic performance of micro-organisms in combination with mass exchange via the tubular system for temporary body insertion described before. Present invention also provides uses of the afore-mentioned device as extracorporeal detoxification system.

## Description

### FIELD OF THE INVENTION

The present invention is related to a tubular body fluid mass exchange system which in principle is composed of a tube arrangement with up to 1.000 tubes, wherein each tube either is a microporous tube with pores in the range of 0,01 to 0,5 µm or each tube has at least one mass exchange aperture with a diameter between 0,1 to 3 mm; these tubes are arranged in a way that they comprise at least one collective inlet and at least one collective outlet, in a preferred embodiment one collective inlet leading to apertures in the environment and one collective outlet leading from apertures in the environment to a collective outlet. This system enables mass exchange in the abdomen via the serosa surface and thus extracorporealdetoxification, including albumin dialysis, and regulation. Furthermore, present invention is related to a mass exchange device which comprises a bioreactor for growing and exploiting the metabolic performance of micro-organisms in combination with mass exchange via the tubular system for temporary body insertion described before. Present invention also provides uses of the afore-mentioned device as extracorporeal detoxification system.

### BACKGROUND

Liver support enables a temporary liver disease treatment to temporary replace liver activity with an extracorporeal device which provides some liver functions. Such a device can be an artificial liver support system, e.g. basing on albumin dialysis, or a bioartificial -liver cell containing- bioreactor.

Liver support is of interest for acute liver failure, e.g. due to virus hepatitis or toxins; to bridge until the organ recovers by regeneration. It is of interest for acute-on-chronic liver failure episodes; to bridge to regeneration; to provide liver function after major surgical liver resection for tumor surgery; to bridge the waiting time for organ transplantation, for bridging to transplant function after initial transplant underfunction and for bridging to donor organ function after small-for size split liver transplantation or related purposes.

An effective treatment for liver disease is the transplantation of a donor organ. This therapy, however, has limitations of organ availability and the patients organ with its potential for regeneration is removed, while the patient needs life long immunosupression.

Hepatic cell transplantation is thought to address liver diseases by additional administration of hepatic cells. Due to a limited half live of the available cell populations and due to immunologic injuries to the transplanted cells, this method is often regarded as temporary therapy. Then, however, the remaining cells may pose a problem for the patient.

Artificial liver support with physical detoxification systems been proven to be partially effective. E.g. the use of the blood plasma carrier protein albumin in so called albumin dialysis liver detoxification systems, including the molecular adsorbent recirculation system (MARS), has provided an interesting concept for the transfer of liver disease relevant factors, toxins and metabolites via extracorporeal hollow fiber membranes with comparable low molecular weight cut off with less than MW 20.000.

A natural semipermeable membrane in the patient body is the peritoneal serosa epithelium, an inner surface of all abdominal organs, including abdominal wall, diaphragm wall, intestines, omentum and other solid abdominal organs and blood vesels. This surface is used therapeutical for peritoneal dialysis, where liquid and electrolyte balance, detoxification and pH regulation is performed by abdominal perfusion with peritoneal dialysis catheters.

Thus, the function of the extracorporeal hollow fiber detoxification and/or mass exchange membranes can be replaced by the natural serosa membrane.

The liver basically regulates substance content in the blood plasma, which is the liquid component of the blood with the blood cells of the body circulation. The plasma of the blood which flows between portal vein and liver veins is metabolized and the content of mass substances kept or regulated at an physiologically equilibrium before it flows again through the body.

Extracorporeal mass exchange to replace liver function is therefore dependent on sufficient mass exchange principles.

Since the mass exchange occurs via the blood plasma, the mass exchange can be performed with plasma of the patient only. Since the blood plasma circulates through the entire body, it could be also metabolized or kept at an equilibrium at any place, e.g. outside of the body.

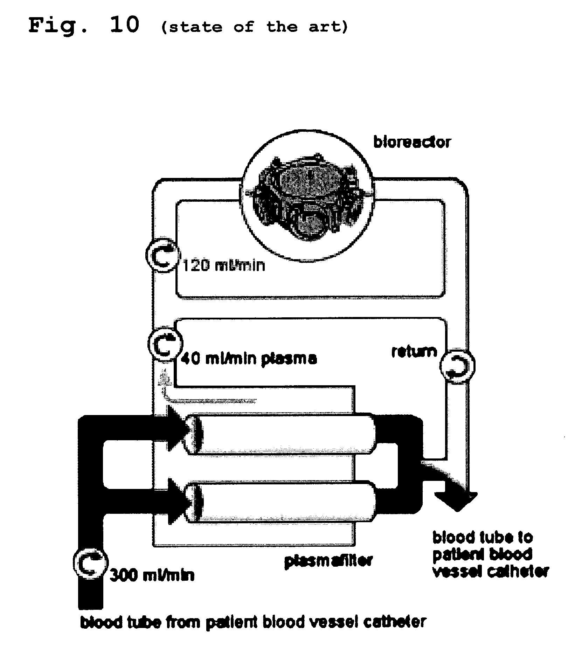

For extracorporeal liver support, mass exchange in the state of the art is performed via direct blood flow using blood catheters in the body vessels, with a pump and blood tubes, through the extracorporeal liver support system and back to the patient (see fig. 10). The liver system typically contains a plasma separation function to separate blood cells from the plasma. Thereby the blood cells cannot clot the liver support system. However, the plasma separation function requires anticoagulation drug treatment to prevent blood clotting in the artificial components which come into contact with the blood cells. Here, the blood cells are injured and cause thrombus complications in the extracorporeal system. Long term application is not possible for more than a few hours, since blood clots (thrombus formation) close the system tubes. It would be sufficient, however, to only circulate the plasma through the extracorporeal system.

To reduce thrombus formation, most systems in the state of the art include a plasma separation step, where a plasma filter (plasma filtration, plasmapheresis) separates plasma out of the bloodstream within the extracorporeal pump / tube circuit (see fig. 10) and feeds it separately to the blood cells through the liver support device. The key element is semipermeable membranes in the plasma filter which allow a mass exchange via plasma flow through the membrane walls while the blood cells are retained.

All these methods require anticoagulation (e.g. with the drug heparin), which limits the application itself to a few days and has side effects including bleeding complications.

While state of the art treatment is feasible because plasma filter membranes separate the blood cells from the plasma, since plasma flow through the system allows longer term use than whole blood flow, the problem is that plasma filters still have to be used outside of the body and require blood flow through artificial tubes/ surfaces and anticoagulation.

By using the natural abdominal serosa membrane in the body, a replacement of the artificial plasma separation membranes is possible, avoiding blood cell damage and reducing the need for anticoagulation.

Material exchange apparatuses, e.g. bioreactors, cell perfusion devices or in general modules are known particularly in the field of liver support systems, , for regulating disturbed substance levels in the body, as an alternative method for animal tests, or for producing biological cell products.

DE 38 37 226 describes a perfusion apparatus for culturing and obtaining liver cells/ hepatocytes. This perfusion apparatus is characterized in that it contains a chamber with a cell compartment and a perfusion compartment, which are separated by a semipermeable membrane. The hepatocytes are immobilized in the hepatocyte compartment. Additional hollow fiber membranes in the cell compartment may drain/ contain bile.

US 3,734,351 describes a method for the liverspecific treatment of blood and a corresponding apparatus. The apparatus includes a chamber with three independent, superimposed and not interwoven compartments formed by two flat membrane inserts in the chamber. The first compartment contains blood, whose constituents can pass through the first membrane layer into the second compartment. The second compartment contains liver cells which metabolize the blood constituents. A second membrane subdivides the second compartment from the third compartment, which contains a dialyzate fluid. Into the third compartment materials are diffused from the cell or second compartment, which can be conveyed out of the same.

The material exchange between the cells and the blood or medium compartment is obtained in the prior art by diffusion. However, this has a disadvantageous effect on the cell metabolism and its controllability. As a result of the design resulting from the fact that the medium flows in on one side and, after diffusion through the membranes, passes out again, there is a different contribution for the cell substrate exchange between the medium inflow and outflow. In the initial area an increased substrate exchange occurs, but this decreases as the medium flows out through the hollow fiber membranes. Thus, the cells are exposed to a different contribution with respect to the substrate and metabolite exchange. Consequently there is no uniform substrate exchange and e.g. the metabolism of the cells and the viability thereof is significantly impaired.

French patent 2 660 323 also discloses a cell culturing module, which contains three independent membrane systems, which are once again spaced in such a way that there are significant material gradients with respect to the supply and disposal.

All these devices are used with extracorporeal blood cell perfusion between the blood via artificial surfaces.

Problems of extracorporeal liver support include therefore
- limited longer term application over a few hours only
- bleeding danger due to use of extracorporeal systems make the devices high risk devices
- bleeding complications due to anticoagulation drugs
- blood cell injury due to extracorporeal artificial blood contacting surfaces
- blood cell mediator/cytokine activation on artificial surfaces with consequences for other organs
- plasmapheresis centrifuges and plasmafilters have to be exchanged every three to eight hours and are expensive
- loss of blood cells with filter changes
- blood access catheters cause complications in the blood vessels.

These state of the art methods limit the application of liver support bioreactors to a few hours, while being labour intense. Mass exchange by whole blood perfusion is limited by the available blood volume flow per minute. Increasing the blood flow to therapeutic levels requires intense patient monitoring, catheter access monitoring and can be associated with the risk of letal bleeding complications. The idea of the invention is to avoid blood catheters, blood removal, blood flow, anticoagulation, plasmafilter/centrifuge, and blood contact with artificial surfaces by using the patients microvascular blood capillary system of the inner abdominal surfaces and the peritoneal serosa membrane as mass exchange membrane.

The peritoneum allows transfer of small molecules. In the ascites, the natural fluid of the abdomen, substances pass the peritoneal serosa, but blood cells can not pass. These abilities are already used for peritoneal dialysis. The peritoneal serosa can thus be compared to a dialysis membrane. Artificial liver support with albumin dialysis has been shown that the presence of the natural carrier protein albumin on dialysis membrane surfaces enhances the transfer of liver failure relevant molecules.

According to the above discussed draw-backs of extracorporeal liver support, it is therefore object of the present invention to provide a mass exchange system which allows a different exchange between extracorporeal support devices and the blood/plasma in the human body, e.g. for detoxification of fluid in the human body. Furthermore, it is object of present invention to provide a mass exchange device that allows extracorporeal mass exchange between body fluids and, e.g., a temporary extracorporeal liver support bioreactor for detoxification and regulation.

This object is, as far as the tubular mass exchange system for detoxification is concerned, solved by the features of claim 1, as far as the mass exchange device for liver support is concerned solved by the features of claim 10. Claim 18 defines uses of the mass exchange device according to present invention. The dependent claims are related to advantageous embodiments, respectively.

According to present invention, a tubular body fluid mass exchange system is provided, which is exposable to an environment, wherein the environment defines a first independent compartment for mass exchange in the body, especially in the abdomen, thus allowing a mass exchange via the serosa surface with the blood, comprising at least one further second independent compartment, which is composed of a tube arrangement with up to 1000 tubes, each tube comprising either a microporous tube/capillary wall with pores in the range of 0,01 to 1,0 µm, preferably 0,01 to 0,5 µm, or at least one mass exchange aperture with a diameter between 0,05 to 3 mm, preferably 0,1 to 3 mm, wherein the at least one further second independent compartments exhibits each at least one main collective inlet. This second compartment (tube arrangement or tubular system) can be constructed in a way that it comprises a main inlet but no main outlet (i.e. only the pores of the tubular system are used for the mass exchange.

The second compartment can however as an alternative embodiment exhibit a main inlet and a main outlet thereby allowing the simultaneous feeding and/or delivery of substances into the tubular system and exchange of these substances with the environment via the pores and at the same time the discharge of different substances from the environment.

A collective main inlet or a collective main outlet is to be understood in a way that after the collective main inlet or before the collective main outlet, the tubular system ramifies into the different single tubes or the tubes of the system are brought together, respectively. Therefore, a plurality of individual tubes of the tubular system can be supplied via only one inlet and/or outlet.

Especially, preferred tubes or capillaries have a total diameter between 0,1 and 3 mm, more preferred between 0,8 and 1,2 mm.

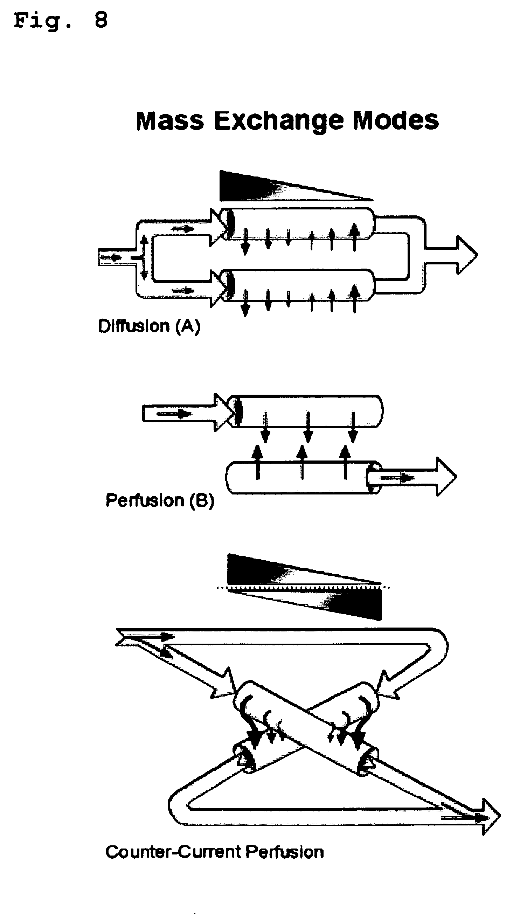

The possible treatment with using the invention bases on the principle of replacing the extracorporeal plasma filter membranes by the use of an intracorporeal natural body surface membrane, the surface of the entire inner abdominal serosa membrane. This locates the mass exchange surface inside of the body and avoids any artificial material contact with blood cells (see fig. 8). Then, a blood cell separation is not required, since the mass exchange occurs via the serosa membrane into the abdominal liquid, the abdominal fluids (ascites) and only these flow through the extracorporeal liver support system. It is well known that the ascites contains the same mass composition of smaller, liver relevant, molecules, which is due to the fact that the serosa epithelium allows mass exchange from the blood flow underneath it via the serosa into the abdominal liquid and back. Instead of flowing blood plasma - as in the state of the art- the invention bases on the flow of ascited through the liver support system.

A combination with the principles of albumin dialysis enhances mass exchange via the abdominal surfaces, therefore the invention can be used in combination with enrichment of the abdominal fluids (the perfusate) with plasma proteins, including albumin.

Consequently, much longer application times are enabled, with reduced anticoagulation and no blood cell flow outside the blood vessels.

In this respect, the invention describes an innovative medical mass exchange principle and a fluid/mass exchange device as an alternative to conventional mass exchange, e.g. to replace blood perfusion/plasmaseparation/plasmapheresis for extracorporeal artificial and bioartificial liver support operation.

According to a preferred embodiment of present invention, the at least one further second independent compartment is composed of 2 to 500, preferably 2 to 100, parallel aligned tubes.

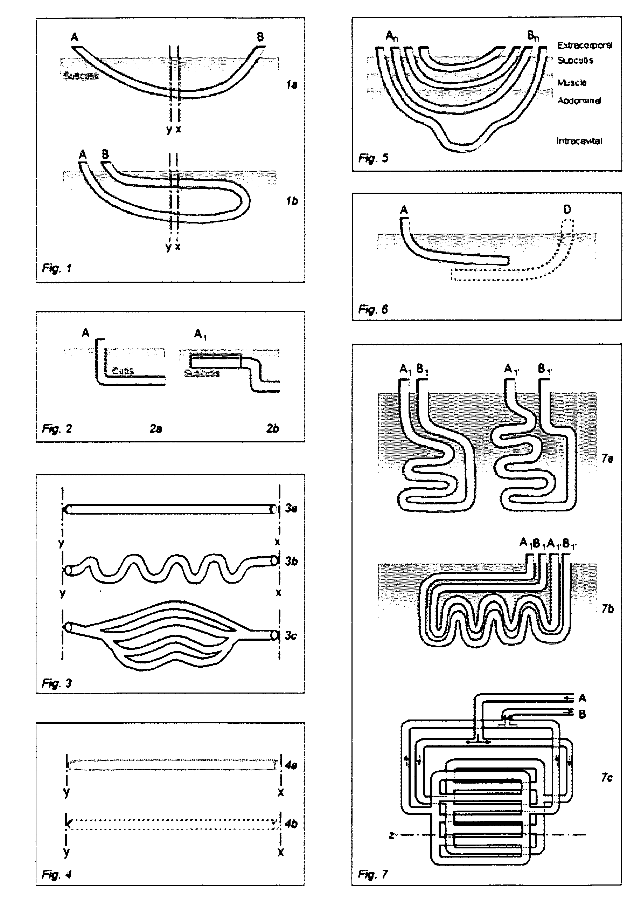

In an additional preferred embodiment of present invention, the at least one further second independent compartment is composed of 2 to 500, preferably 2 to 100, meander-like aligned tubes. In this embodiment, the tubular body fluid mass exchange system is composed of two separate tubular systems, wherein the first tubular system comprises a collective main inlet and the other one a collective main outlet. Examples for this embodiment are given in figure 7. In detail, this embodiment of present invention can be operated in perfusion mode, i.e. a fluid is introduced into the second compartment and perfuses the porous part of the tubular system, whereas the third compartment operates in opposite direction, i.e. fluid enters through the porous part of the porous tubular system and can be discharged via the collective outlet.

Furthermore, it can be advantageous if three further, a second, a third and a fourth independent compartment are comprised, which are each composed of a tube arrangement with up to 500, preferably 100 tubes. This embodiment is an extension of the inventive concept as described above.

As a further advantageous embodiment of the inventive concept, at least two further independent compartments, a second and a third compartment, are comprised, wherein each independent compartment is composed of a tube arrangement, which comprises at least one main inlet and at least one main outlet, preferably at least one collective main inlet and at least one collective main outlet. This embodiment is especially preferred, when at least two independent compartments, of which each is composed of a tubular system, is comprised; these compartments exhibit each a main inlet and a main outlet, respectively. According to this concept, the operation of the tubular system, which is composed of at least two compartments, in perfusion mode, is possible.

As an alternative advantageous embodiment, the second compartment comprises at least one main inlet but no main outlet whereas the third compartment comprises at least one main outlet but no main inlet. The second and the third compartment can be aligned and are operated in open perfusion mode. This special embodiment uses the second compartment (comprising the main inlet) as compartment for the introduction of substances, whereas the third compartment (outlet) is used to deduct substances from the environment.

A further advantageous embodiment provides that the tubes are composed of medical grade tubes for temporary implantation, including silicone rubber, polyvinylchloride, polyurethane and polyethersulfone.

In a further aspect, present invention is related to a mass exchange device, comprising
a) at least one afore-mentioned tubular system,
b) at least one module (also referred to as bioreactor) for growing and for exploiting the metabolic performance and/or for obtaining microorganisms, in particular for cells, composed of an external housing, at least two, preferably three independent membrane systems, at least one, preferably at least two of which is/are designed as hollow-fibre membranes and arranged in the interior of the module, these hollow-fibre membranes forming a densely packed spatial reticulate structure and micro-organisms, which are located in the interstices of the reticulate structure and/or adhere to the hollow-fibre membranes, in which the reticulate structure is composed of intersecting and/or superimposed hollow-fibre membranes and is constructed in such a way that the substrate supply and substrate disposal conditions for the microorganisms are virtually identical at any given place in the interior of the module, wherein the module features at least one inlet and at least one outlet,
c) at least one feed line, connecting the at least one outlet of the at least module with the at least one inlet of the at least one tubular system,
d) at least one feed line, connecting the at least one inlet of the at least one module with the outlet of the at least one tubular system, and
e) at least one pump being aligned in the at least one feed line and/or the at least one feed line.

As an optional element f), the device can comprise as advantageous embodiment a filter to collect mass aggregates, such as protein flakes etc. thus avoiding a clogging of the membrane systems of the bioreactor.

In general, the mass exchange device according to present invention is composed of the afore-mentioned tubular system and a bioreactor, which allows the regeneration e.g. of abdominal fluids. This reconditioning of the body fluid is accomplished by microorganisms which are located inside the reticulate structure of the bioreactor or module, respectively.

According to the invention the term module is understood as a material exchange apparatus in form of a three-dimensional body, which includes an outer casing and an inner space which comprises a close packed space network. The module shape can vary widely. The module can e.g. have a rectangular or n-angular layout. Spherical or disk-shaped modules are also suitable for the purposes of the invention.

It is essential to the invention that use is made of at least two independent membrane systems, at least two independent membrane systems being constructed as hollow fiber membrane systems and the latter form in the inner area a close packed network.

A first independent hollow fiber membrane system is used for the medium inflow. A second independent hollow fiber membrane is provided for the supply of micro-organisms, e.g. with oxygen, or removal of CO₂. According to the employed mode of medium flow, an outflow can be ensured by a third independent membrane system. Alternatively, two independent medium membrane systems can be operated in cross-flow counter current operation.

Each of the individual independent hollow fiber membrane systems comprises a plurality of individual hollow fiber membranes and in each case the hollow fibers of a system communicate with at least one inlet or an inlet and an outlet. This ensures that the hollow fibers of any particular independent system can be simultaneously supplied via the inlet, e.g. with medium.

In the inner space of the module the independent hollow fiber membrane systems form a three-dimensional, close packed network in such a way that at virtually any point of the network a few micro-organisms have virtually identical conditions for substrate supply. This leads,to a substantial simulation of the conditions in the physiological organs with their own arteries and veins, such as e.g. the liver, with the arrangement of hepatocytes in lobuli. As a result of the independent arrangement of the different membrane systems the module offers the advantage of a decentral transport of nutrients, synthesis products and gases to and from a plurality of micro-organisms independently of the position thereof in the module, as is the case in the cell environment in natural organs.

According to the invention the medium outflow is ensured by the third independent membrane system. This membrane system can also be a hollow fiber membrane or a changeable flat membrane or a changeable capillary membrane. What is decisive is that the third membrane system is also independent of the two other hollow fiber membrane systems.

A preferred embodiment proposes that the close packed network in the inner area is formed by three independent hollow fiber membrane systems. In this case all the independent membrane systems are hollow fiber membranes located in the inner area. The first independent hollow fiber membrane system is used for medium inflow, the second for medium outflow and the third for additional supply, e.g. with oxygen. The close packed network then comprises these three independent systems.

The close packed network can have different constructions, provided that it is ensured that in each case few micro-organisms in the inner area have an identical substrate supply. The spatially close packed network can e.g. comprise close packed layers, in each case independent system layers alternating. A first layer comprising individual hollow fiber membranes is positioned horizontally. The second layer once again comprising individual hollow fiber membranes is positioned in the same plane, but rotated with respect to the first layer, e.g. at an angle of 90°. These layers alternate and together form a close packing system. The third independent hollow fiber membrane system, which once again comprises individual layers of hollow fiber membranes, traverses the said two layers, e.g. vertically from top to bottom and consequently "interweaves" the first two independent layers.

According to a further development of the invention, three independent hollow fiber membrane systems are so superimposed in alternating layer form, that all are positioned in one plane, but are in each case rotated by e.g. 60°.

This close packed network is placed in the inner area of the module. Due to the fact that each independent system communicates with at least one inlet or an inlet and outlet, it is ensured that the medium supplied reaches all locations in the module in a uniform manner and in the same way a uniform oxygen supply is obtained. Due to the third independent system for the medium outflow, continuously and uniformly the medium can be removed from all points of the entire module.

According to a preferred embodiment, in addition to the three hollow fiber membrane systems in the inner space, use is made of a further independent membrane system for the medium outflow. For this purpose, according to the invention either a replaceable flat membrane or a replaceable capillary membrane is fitted on the outer casing. This ensures that there is a problem-free medium outflow, even over long periods of time.

According to another development of the invention, the close packed network is formed by two independent hollow fiber membrane systems, a first being used for the medium inflow and a second for oxygen supply and the third independent membrane system is constituted by a replaceable flat or capillary membrane, which is fixed to the outer casing and is used for the medium outflow. The close packed network in the inner area and which is formed by the two hollow fiber membrane systems is constructed in the same way as described hereinbefore.

According to the invention, as hollow fiber membranes preference is given to the use polypropylene, polyamide, polysulphone, cellulose or silicone. The choice of the hollow fiber membranes is a function of the molecules provided for the material exchange. However, according to the invention, it is possible to use all standard hollow fiber membranes, which are known from the prior art for material exchange apparatuses.

According to the invention, when using three independent hollow fiber membrane systems, which in the inner area form a close packed network, a capillary system of liquid-impermeable capillaries, such as e.g. of refined steel or glass can be used. This can then be used for the thermostatic control of the module with its inner area. It also permits a uniform cooling of the module with its inner space and introduced micro-organisms to below -20 DEG C. According to a further development of the invention, it is also possible to use further hollow fiber systems for thermostatic control or for cooling to below the freezing point.

A further preferred development proposes that the outer casing is formed by a sealing compound in which the capillaries are cast. The outer casing cast in such manner that there is an access from the outside into the volume of the capillaries.

According to a further development the module has different accesses which provide access to the module. A first access into the internal area of the module serves for introducing the micro-organisms into the module, whilst other accesses are used for the pressure, pH and temperature measurements within the inner area of the module.

The invention also relates to a process for operating the module. According to the invention the inner area of the module is filled with micro-organisms. Following on to this the physiological conditions necessary for culturing and/or obtaining are set. The medium is fed into a first independent hollow fiber membrane system, passes through the hollow fiber membrane and metabolizes with the micro-organisms, which are either located in the cavities or adhere to the membranes. The material exchange between the microorganisms and the medium takes place by convection. In order to ensure this, it is merely necessary for the outlet on the independent hollow fiber membrane system into which the medium is supplied to be closed, so that the medium must pass through the hollow fiber membranes. However, the module according to the invention can also be operated in the diffusion operating mode, in that the outlet for the medium flow in the independent hollow fiber membrane system is left open and consequently there is only a diffusion operation. The particular advantage of the invention is that as a result of convection operation there is an optimum, controllable substrate exchange, whereas in the prior art there is only a material exchange by diffusion.

The invention offers the further advantage that the hollow fiber membranes or capillaries can be coated with a substrate suitable for the cells or microorganisms in order to provide micro-organism adhesion to the capillary systems.

A further advantage of the invention is that in addition to the cell type used, one or more other cell types can be used in accordance with a co-culture procedure. These additional cell types can be cultured in the lumen of one or more independent capillary systems. Cells for co-culturing are e.g. nonparenchymal cells from liver sinusoids.

The module according to the invention offers to the further advantage that one of the independent membrane systems can be used for conveying away e.g. bile constituents.

According to a further advantageous variant, one of the independent membrane systems is used for dialysis.

As a result of the proposed independent membrane systems, there is a considerable scope for very varied uses of the module.

According to a particularly preferred embodiment the micro-organisms are cells and in particular liver cells.

According to present invention, the above-mentioned device can be used for medical purposes, especially as apparatus for medical purposes, especially as apparatus for extracorporeal liver support, for liver detoxification including for albumin dialysis, for assisting the natural liver regeneration, for bridging to liver recovery after liver surgery, for bridging to liver transplant function after initial transplant under-function, for bridging to donor liver function after small-for size split liver transplantation, for dialysis, for drug, mediator and/or factor admission and/or for drug, mediator and/or factor regulation.

Furthermore, present invention provides a method for mass exchange with an environment, which forms a first independent compartment with a tubular mass exchange system according to the invention by perfusing a liquid into the mass exchange system via the at least one inlet and performing a mass exchange via the pores of the system. Preferably, the first compartment is the abdomen, i.e. the mass exchange system is aligned inside the abdomen, which can be accomplished by endoscopical surgery, among others.

Especially preferred is, if more than one second compartment, i.e. at least two tubular systems, are aligned inside the first compartment and operated in counter-directional mode, i.e. one compartment is used for the introduction of liquids, the other one for the deduction.

### BRIEF DESCRIPTION OF DRAWINGS

The invention is described in greater detail hereinafter relative to non-limitative embodiments and the attached drawings, wherein:
FIGS. 1 to 7 illustrate different possibilities of aligning tube catheters for medical purposes inside the human body.
FIG. 8 describes different modes of operation of tubular systems.
FIG. 9a-c depict mass exchange devices comprising a tubular system and a bioreactor. FIG. 9a and 9b describe two similar embodiments, whereas the arrangement of the mass exchanger tubes in the abdomen differ. FIG. 9c illustrates the advancements from the state of the art (left) to the use of the invention (right) in terms of minimising complexity.
FIG. 10 demonstrates the principle of extracorporal liver support as known from the state of the art.
FIG. 11 is a cross-section through a module of the mass exchange device having a rectangular shape.
FIG. 12 shows individual hollow fiber membranes, corresponding to FIG. 11, of three independent membrane systems.
FIGS. 13A, 13B, 13C, 13D, and 13E show independent hollow fiber membrane systems according to FIG. 11 with cell cultures.
FIG. 14 shows individual capillaries of four independent systems.
FIG. 15 is a perspective view of a plane of a module of the mass exchange device with four independent hollow fiber membrane systems according to FIG. 14.
FIG. 16 illustrates three possibilities of medium outflow from a module of the mass exchange device.
FIG. 17 illustrates alternative arrangements of the membrane heads on one side of a module of the mass exchange device.
FIG. 18 is a sectional view through a module of the mass exchange device with hollow fiber membranes and membrane heads with different types of hollow fiber membranes.
FIG. 19 is a fragmentary sectional view showing different accesses to the module of the mass exchange device.
FIG. 20 is a schematic view illustrating an exemplified use of a module of the mass exchange device as an extracorporeal support system.

Figure 1 describes a tube or catheter, being inserted from the outside of the body (upper part) through the skin ("cutis") through the tissue under the skin ("subcutis") into the body and led outside of the body again. The beginning (A) and the ending (B) of the tube remains outside of the body. If this tube exhibits permeable walls or is perforated, mass exchange from out an outside of the body arranged extracorporeal devices to/from the body is possible. Fig 1a and 1b describe variations of the arrangement of the in- and outlet. Lines y and x refer to figures 3 and 4, where between x and y additional tube is extended.

Figure 2 describes a variation of the beginning (A) or the ending (A1) of the tubes in fig. 1. Fig. 2a describes the situation of fig. 2, while 2b describes the use of a so called port which is implanted into the subcutis and exhibits a rubber-like membrane. To enable temporary access to the lumen of the tube, another tube with medical injection needle is injected through the skin and the rubber membrane and connects the tubes. When the needle is not used, the lumen of the tube in figure 1 remains sterile in the body.

Figure 3 describes variations of the tube in fig.1 being a simple extension of the tube (upper, 3a), irregular shapes (middle, 3b) or branching out an in as in a body capillary vessel system (lower, 3c).

Figure 4 describes that the part of the tube in fig.1 which lies within the body may exhibit a porous permeable wall (upper, 4a) or a perforated wall (lower, 4b).

Figure 5 illustrates how deep the tube of figs. 1,3,4 can be implanted into the body: End (Bn) and beginning (An) lie outside of the body and the middle part (see figs 3,4) lies either in the subcutis, in a muscle, in the abdominal cavity, or may lead though all these structures into a lumen of an abdominal organ, such as an intestine and out.

Figure 6 describes that the tube beginning (A) does not need to continue to the end (D); if the tubes are porous or perforated (see fig. 4), a perfusion of liquids can allow mass exchange with the body fluids. The lumen of tube A comprises a first compartment, the body fluids a second compartment and the lumen of tube D a third compartment. Mass exchange can occur from A to D via all compartments.

Figure 7 shows an elaborated version of the principle described in fig. 6: in fig.7a, the liquid in the first permeable compartment can recirculated from A1 to B1 and the liquid in the second permeable compartment can be perfused from A1' to B1', while liquid from A1/B1 can be perfused via the body tissue compartment to A1'/B1'. Also, a counter-current (or counter-directional) perfusion and thus mass exchange in the two circuits is possible. The latter is improved by using the configuration 7b for counter-current flow operation. 7c explains an even improved version of the mass exchange fluid perfusion devices described in 7a and 7b.

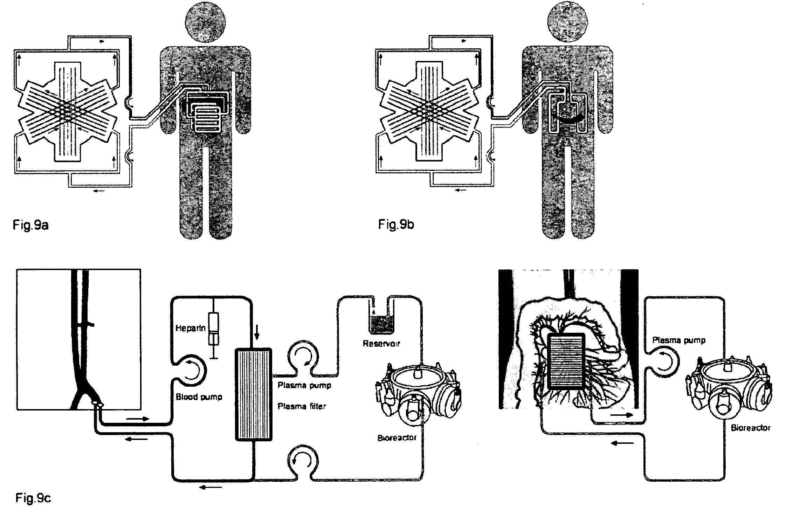

Figure 8 shows mass exchange principles in permeable tubes with mass exchange mostly via diffusion (this compares to fig. 3c), via perfusion (this compares to figure 6), and via counter-current flow (or counter-directional flow) operation (this compares to figure 7, and to fig. 9).

Figures 9a-c illustrate how the device of figure 7c can be implanted into the abdominal cavity and connected to an extracorporeal system. In figure 9a, the device is placed in the abdomen (left), and tubes leading to and from that implanted device are connected to an extracorporeal metabolic device, e.g. a bioreactor for temporary liver support (right). Figure 9b describes a similar embodiment as in figure 9a, whereas the arrangement of the mass exchange tubes in the abdomen differ. In the illustrations, the bioreactor (left) operates also with a multicompartment mass exchange principle using counter-current flow operation. The figure explains how extracorporeal liver support would be used, if the bioreactor on the left would contain liver cells. This total assembly is referred to as mass exchange device according to present invention. Figure 9c shows a similar embodiment and is used to illustrate the advancements from the state of the art (left) to the use of the invention (right) in terms of minimising complexity.

Figure 10 demonstrates the principle of extracorporal liver support according to the state of the art, wherein mass exchange in the state of the art is performed via direct blood flow using blood catheters in the body vessels, with a pump and blood tubes, through the liver support system and back to the patient (see fig. 10). Here, the blood cells are injured and cause thrombus complications in the extracorporeal system. Long term application is not possible for more than a few hours, since blood clots (thrombus formation) close the system tubes.

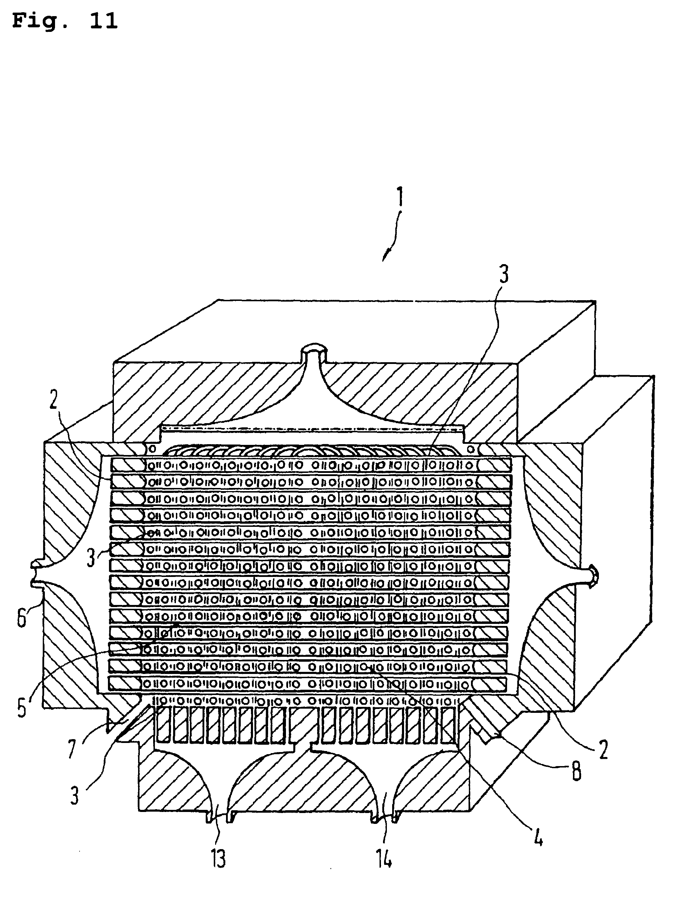

Figure 11 shows a vertical cross-section through a possible module design showing a module 1 having a close packed network 5 of three independent systems. The module 1 includes an outer casing 2 and an inner space 4 housing the close packed network 5. The outer casing 2 comprises a sealing compound in which the capillaries are cast. The outer casing 2 has an access from the outside into the internal diameter of the capillaries. The sealing compound maintains the mechanical stability of the module. The close packed network 5 in the inner space 4 of the module 1 has the following construction.

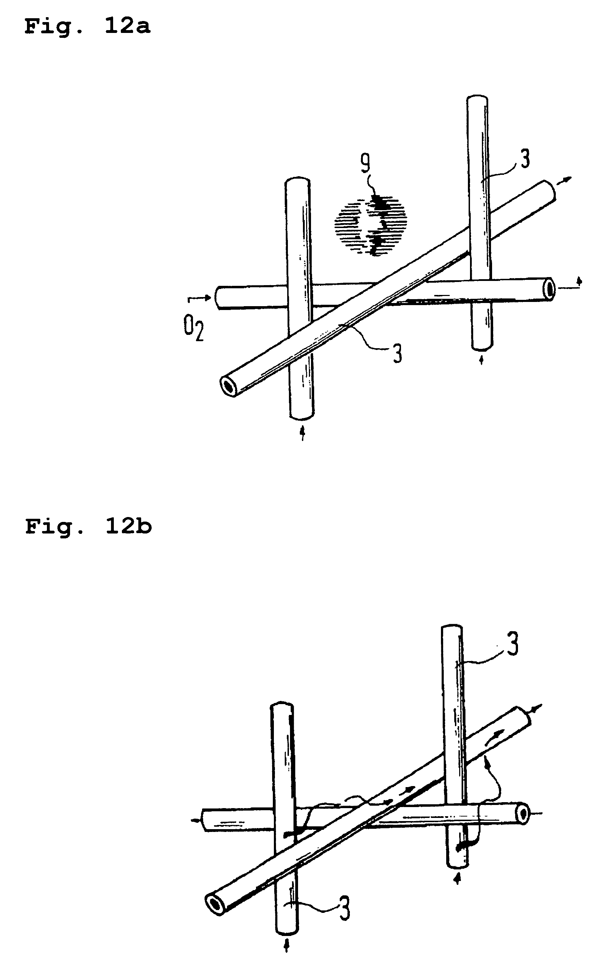

A first independent hollow fiber membrane system is formed by layers of hollow fibers 3, the first layers being positioned horizontally. Each individual layer comprises linear hollow fiber membranes 3, which are closely juxtaposed. The second independent hollow fiber membrane system is also formed by layers, which are turned by an angle of 90° in the same plane, so that layers of the two independent systems alternate. These layers are in part represented by circles in figure 11. These alternating layers are now tightly superimposed in a module 1. In a test module with external dimensions 12x12 cm approximately 100 layers are superimposed. The third independent hollow fiber membrane system is represented by vertical lines in figure 11. These lines represent hollow fiber membranes, which vertically traverse from top to bottom the layers located in a single plane. In the described test module there are approximately 50 layers, which interweave the module vertically from top to bottom. As a result of this design of the close packed network it is ensured that few cells, as can be seen figures 12a and 12b, have an identical substrate supply.

For the operation of the module medium is supplied through the medium inflow head 6 into the internal diameter of the capillaries. The end opposite to the head 6 is closed. This medium now passes through the membranes of the capillaries and there is a material exchange with the cells 9, which are either located in the cavities and/or adhere to the capillaries. The cells 9 are supplied with oxygen during the material exchange. The oxygen passes through the oxygen inflow head 13 into the internal diameter of the capillaries and is removed via the oxygen outflow head 14. In the present case the hollow fiber membranes 3 of the independent system for the oxygen supply are formed by U-shaped hollow fiber membranes. The conveying away of the material takes place via the third independent hollow fiber membrane system through the membranes indicated by the circles. These hollow fiber membranes then issue into a medium outflow head 15, from where the medium can be removed (not shown).

Cells 9 can be supplied through the access 7 into the inner area of the module 1. A further access 8 is provided for carrying out measurements in the inner area of the module, such as e.g. pressure, temperature or pH measurements.

The described module construction can be varied at random. The module shape need not be rectangular and can instead equally well be n-angular or some other random hollow body, provided that the hollow body makes it possible to house in an inner space 4 a close packed network 5 of independent hollow fiber membrane systems. What is essential to the invention is the design of the close packed network 5 of the hollow fiber membrane system. Diverging from the possibilities shown in figure 11 it is equally possible to superimpose in close packed manner individual layers, each individual layer being formed from alternating, independent hollow fiber membranes. According to another possibility the first two layers are at an angle .alpha. of 0 to 180 DEG, instead of 90 DEG. Random possibilities also exist for the third independent hollow fiber membrane system, provided that it is ensured that there is an almost identical substrate supply both for the medium supply, the oxygen supply and the medium outflow.

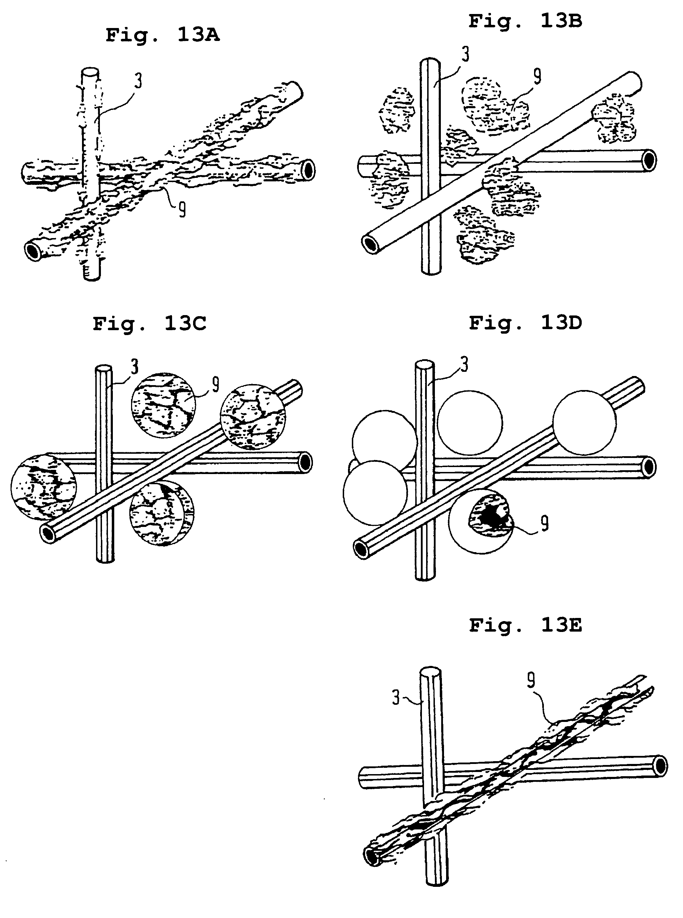

Figure 12 shows individual hollow fibers 3 of three independent hollow fiber membrane systems. Figures 13A and 13B show a random point from the inner space 4 of the module 1. Figures 12a and 12b reveal the essential concept of the invention. As indicated by the arrows, medium is supplied through the hollow fiber membrane 3, which is a single membrane from a first independent membrane system, said medium passing through the membrane 3. The microorganisms 9, here in the form of cells, are located in the cavities of the close packed network 5 and/or, as shown in figure 13, adhere to the capillaries. A material exchange takes place between the cells 9 and the medium. According to the invention, through a further independent hollow fiber membrane system, a single membrane 3 being shown in figures 12a and 12b, the cells are e.g. supplied with oxygen (horizontal membranes). A third independent hollow fiber membrane system, once again only represented by an individual membrane 3, is used for the medium outflow. Thus, the medium is removed after exchange with the cells.

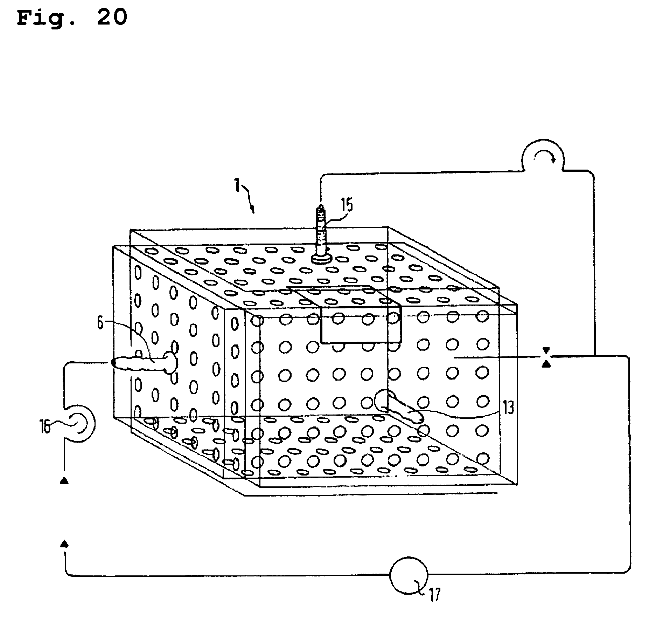

The individual hollow fiber membranes shown in figures 12a and 12b are components of the close packed network 5 in the interior of the module 1 and which is constructed in such a way (figures 11 and 20), that at each point within the module 1, there are identical substrate supply conditions. At each point of the module 1 the cells 9 are uniformly supplied and consequently an optimum material exchange and controllability thereof are ensured. Figure 12b shows the perfusion of the cells 9 and the medium flow along the capillaries. As a result of the design according to the invention the situation is very similar to the true conditions, e.g. in the liver.

Figure 13 shows different cell culture methods within the module. According to the invention the microorganisms 9 can be placed in different ways in the module 1. Figure 13A shows an adhesion culture to the membranes, Figure 13B an aggregate culture between the membranes, figure 13C microcarrier cultures between the membranes, figure 13D capsule cultures between the membranes and figure 13E cocultures in separate membrane compartments. As a result of the inventive construction of the module 1, i.e. through the close packed network in the inner area of the module constituted by individual hollow fiber membranes, it is possible to use different cell culture methods and therefore achieve a greater scope of use for the invention.

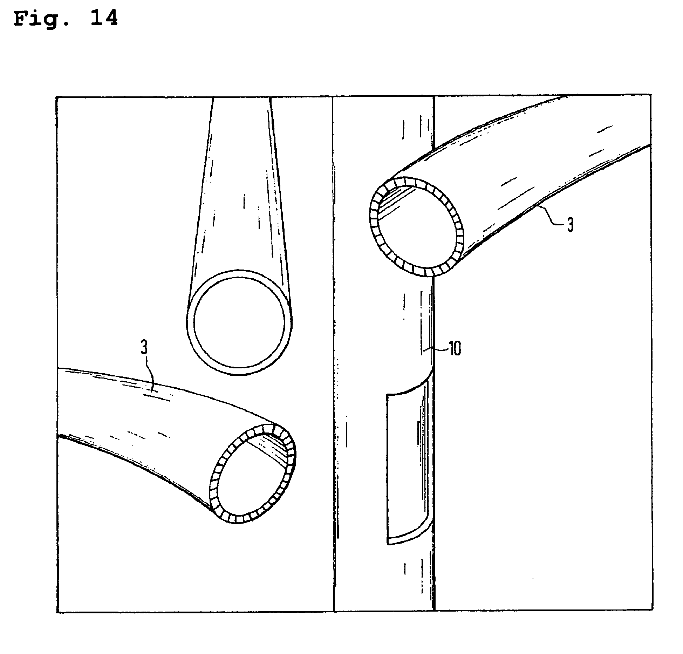

Figure 14 shows individual capillaries of four independent systems and how the capillaries are coated with adhesion factors. There are three systems in one plane. Figure 14 shows four independent membrane systems, which are in each case part of an overall system. As described relative to figures 11 and 12, one hollow fiber membrane system 3 is used for medium inflow, a third system for oxygenation and a further independent system 10 e.g. for dialysis, as a heat exchanger for the lyophilization of cells or for cocultures with further cell types. The black points on the membranes 3 indicate the pore openings (cells not shown). Through the provision of four different independent hollow fiber systems, it is possible to significantly broaden the possible uses of the object of the invention. Apart from the essential advantage of the invention that there is an identical substrate supply for virtually any cell within the module, the invention also makes it possible by coating the capillaries with adhesion factors within the module to house a maximum number of cells, which are virtually all subject to the same substrate supply and therefore have an increased life.

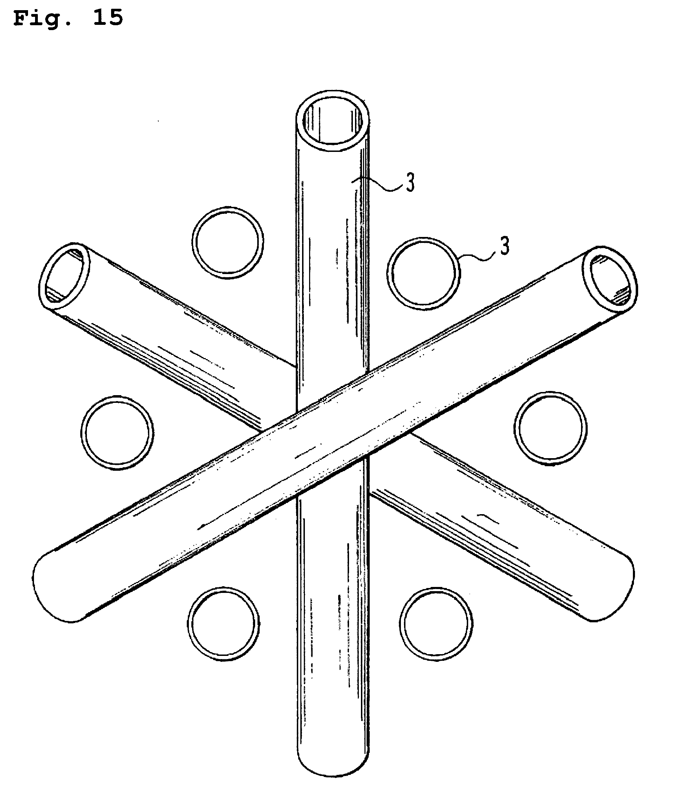

Figure 15 shows in a three-dimensional plan view a plane of a module with four independent hollow fiber systems like figure 14. A first independent system is used for the medium inflow, a second for medium outflow and a third for oxygen supply. A fourth independent hollow fiber system can be used for dialysis or as a heat exchanger for the lyophilization of the cells or for cocultures.

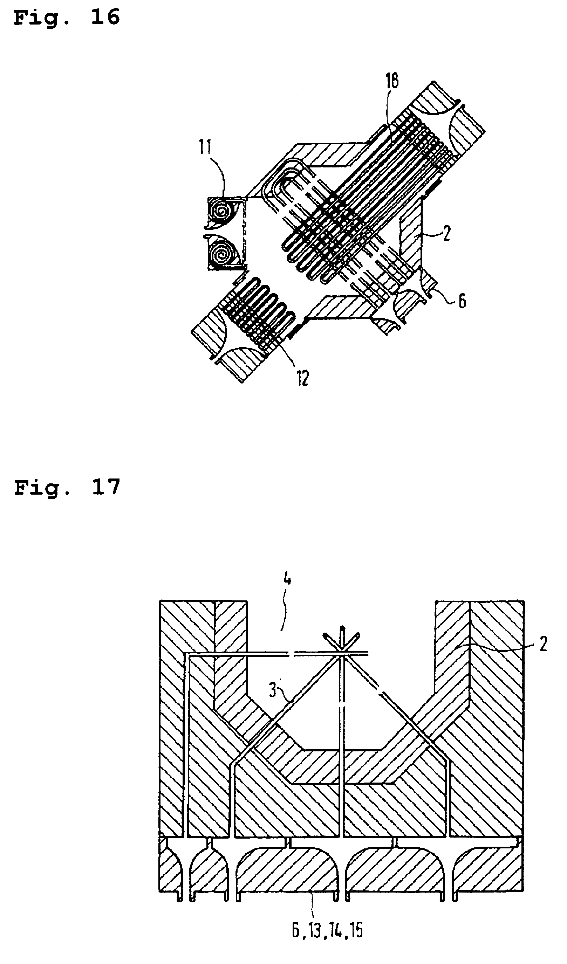

Figure 16 shows different possibilities of the medium outflow from the module 1. In the inventive constructions described relative to figures 11 to 15 the medium outflow has taken place by means of the hollow fiber membranes 3. According to a further development the medium outflow not only takes place through hollow fiber membranes, which are a component of an independent hollow fiber system within the close packed network 5, but also through a number of variants. Figure 16 shows the different possibilities. The outflow either takes place by means of fixed inserted capillary membranes (connected to the outlet head 6) spatially and in a predetermined arrangement with respect to the remaining capillary membranes, or via replaceable capillary membranes 12, which are also spatially independent with respect to the remaining membrane systems. The replaceable capillary membrane 12 is fixed to the outer casing 2, which must have an opening suitable for receiving said membrane 12.

According to another variant replaceable flat membranes 11 are located on the outer casing 2, which must contain corresponding openings. According to a further variant use is made of replaceable capillary membranes 18, which can be drawn out in the case of blockages.

It is decisive that the specific medium outflow devices are independent of the further hollow fiber membrane system. These different medium outflow devices can be supplied in addition to an already present medium outflow via a hollow fiber capillary membrane forming part of the close packed network.

Figure 17 shows a special arrangement of membrane heads 6, 13, 14, 15 on one side of the module 1. This design of the invention, i.e. the arrangement of all the inflows and outflows on the outer faces of the module 1 on a single side offers the advantage that all the connections are in one plane.

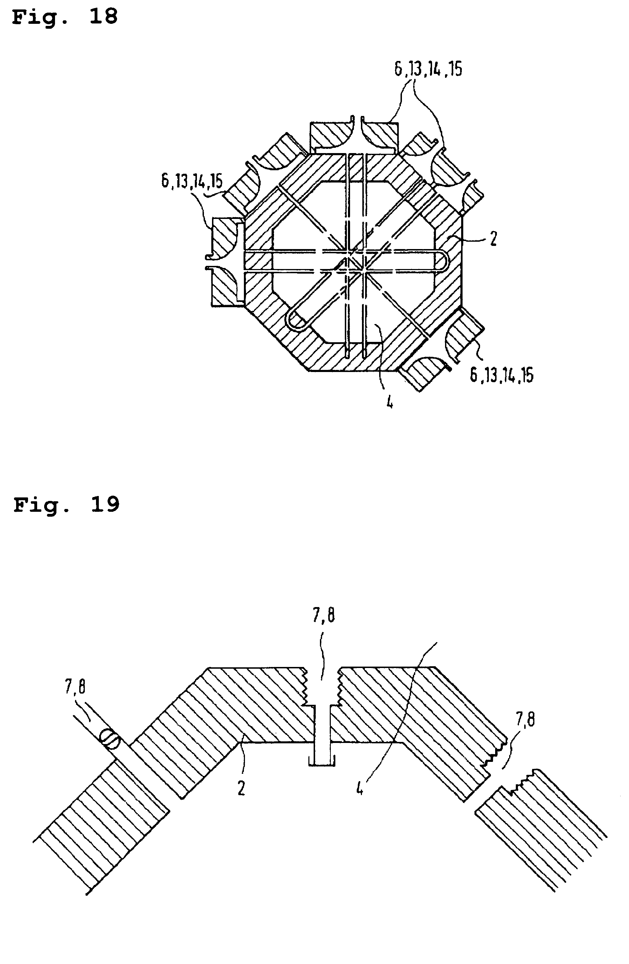

Figure 18 is a cross-section of an octagonal module with capillary membranes and membrane heads 6, 13, 14, 15. Figure 18 once again shows the different possibilities as to how the capillaries can be located in one plane. Thus, the capillaries can be in double-tube form with one head for the medium inflow and outflow. According to another variant one capillary is straight and has a corresponding head for the medium inflow. According to a third possibility there is a single straight capillary with a dead end and a head for medium inflow and outflow. In a fourth possibility there is a double-tube capillary with circulation and a head for medium inflow and outflow.

Figure 19 shows the different accesses in the module casing which provide access to the module inner space 4. Thus, the module can have different accesses 7, 8, in order to carry out measurements for the pressure, temperature, flow, pH or osmolarity.

Figure 20 shows the use of a module 1 as an extracorporeal liver support system. The medium, in this case the blood plasma, is supplied via a blood pump 16 into the medium inflow head 6 of the module. Oxygen supply takes place by means of the inflow head 13. The oxygen is removed again via the outflow head 14 (not shown). The medium removal takes place by means of the medium outflow head 15 and corresponding control and pump units 17.

As a result of the design according to the invention, all the cells in the system have the similar substrate supply conditions. The medium to be supplied is uniformly provided at all locations within the module, as is the oxygen supply. Through the use of different capillaries a selective removal, e.g. lipophilic substrates is possible. As a result of the design according to the invention the life of e.g. liver cells could be increased from 10 days to 7 weeks and the activity increased by 40%.

Those skilled in the art will appreciate that the invention described herein is susceptible to variations and modifications other than those specifically described. It is to be understood that the invention includes all such variation and modifications. The invention also includes all of the steps, features, compositions and compounds referred to or indicated in the specification, individually or collectively and any and all combinations or any two or more of the steps or features.

The present invention is not to be limited in scope by the specific embodiments described herein, which are intended for the purpose of exemplification. Functionally equivalent products, compositions and where appropriate methods are clearly within the scope of the invention as described herein.

Throughout this specification and the claims that follow, unless the context requires otherwise, the word "comprise", or variations such as "comprises" or "comprising", will be understood to imply the inclusion of a stated integer or group of integers but not the exclusion of any other integer or group of integers.

Extracorporeal liver support with bioreactors is in development, using the liver function of hepatic cells. Extracorporeal liver support bioreactors enable a temporary application of hepatic cells, being easily removable after application. The use of semipermeable membranes in a bioreactor enables an immune isolation of then cells from the patient's immune system. These systems base on either extracorporeal whole blood perfusion of the bioreactor, or a plasma separation step with plasma phereses centrifuges or membrane plasma filters, where the blood cells are separated and the bioreactor is perfused with the patients plasma.

A drawback of the use of plasma centrifuges or plasma filters is blood cell interactions with the patient's blood cells. Extracorporeal whole blood perfusion is associated with blood cell damage and loss and requires anticoagulation drugs, e.g. citrate or heparin. Drug application need to be monitored and is associated with the risk of bleeding in other patients organs. Membrane fouling limits the plasma filter function, too. Mass exchange limitations include problems in the bioreactor, the plasma filter or plasma centrifuge, catheter blood access, circulatory blood availability. It would be desirable, to avoid some of these problem areas.

These state of the art methods limit the application of liver support bioreactors to a few hours, while being labour intense. Mass exchange by whole blood perfusion is limited by the available blood volume flow per minute. Increasing the blood flow to therapeutic levels requires intense patient monitoring, catheter access monitoring and can be associated with the risk of letal bleeding complications.

The idea of the device and method described here is to avoid blood catheters, blood removal, blood flow, anticoagulation, plasmafilter/centrifuge, and blood contact with artificial surfaces by using the patients microvascular blood capillary system of the inner abdominal surfaces and the peritoneum membrane as mass exchange membrane.

The peritoneum allows transfer of small molecules. In the ascites, the natural fluid of the abdomen, substances pass the peritoneal serosa, but blood cells can not pass. These abilities are already used for peritoneal dialysis. The peritoneal serosa can thus be compared to a dialysis membrane.

Artificial liver support with albumin dialysis has been shown that the presence of the natural carrier protein albumin on membrane surfaces enhances the transfer of liver failure relevant molecules.

The concept described herein involves:
- using the serosa instead of artificial extracorporeal membranes,
- using the heart pump driven microcaoillary vascular abdominal system instead of extracorporeal blood flow via catheters, tubes and pumps,
- avoiding anticoagulation by keeping the blood cells in the vascular endothelial space,
- use of open porous capillaries/tubes in an arterio- venous-like arrangement for fluid distribution for high flow throughput,
- use of a 3-compartment-arrangement for fluid distribution via counter-current flow in two compartments for enhanced mass exchange,
- using albumin in the perfusate for better mass exchange,
- using alternating pressures/fluid levels for further enhancing mass exchange by better reaching all available surfaces,
- widening the abdominal space by application of all patient fluid substitution during application into the abdominal space,
- further enhancing the fluid level in the abdominal space by additional fluid application in case of good kidney function,
- optionally use of alternating single catheter in-and-out flow techniques as known from single needle dialysis techniques.

Advantages over the state of the art include:
Clinical:
   - No need for drug based anti-coagulation of the patient (e.g. use of heparin injections), as in conventional blood perfusion or plasma filtration/plasma pheresis, reduction in application expenses;
      No injury to blood cells, as in conventional blood perfusion or plasma filtration/plasma pheresis;
   - No membrane fouling of the mass exchange membrane;
   - No attachment of blood cells to artificial surfaces/filter membranes, as in conventional plasma filtration, leading to system clotting and patient blood cell loss;
   - Consequently, longer term clinical applications are possible in comparison to the state of the art.
   - while side effects are avoided for the patient.
Safety:
   - Advancing from potential hazardous extracorporeal blood management to less dangerous abdominal fluid management without danger of bleeding and consequently dead of patients.
Technical:
   - Less equipment, technical devices, reduction in safety control devices (reduction of pumps in the circuits, simpler disposables, reduction of pressure/flow monitoring;
   - Reduction in safety control units;
   - Reduction in staff maintenance time in intensive care units required;
   - Device service/maintenance reduction;
   - Consequently reduction in system expenses and price per treatment.

The tubular body fluid mass exchange system preferably is constructed as temporary implantable device for bioreactor -abdominal fluid exchange via at least two compartments with an artero-venous like perforated tube- or open porous hollow fiber capillary system. The lumen of the abdominal space is compartent three, whereas the lumen of the veinous-like system is compartment one and/or the arterial-like system is compartment two. The veinous-like system and the arterious-like system form the central part of the tube arrangement. They are provided with a collective inlet and outlet. These compartments can be connected via a pump driven circuit for the perfusion of the extracorporeal bioreactor and be operated in counter-directional flow operation with a bioreactor for liver support. In applying the method and/or using the device, bioreactor perfusate for mass exchange between bioreactor and patient is enabled by perfusion of a liquid solution in a circuit connecting the bioreactor and the inner abdomen. Mass exchange is performed by perfusion via the serosa epithelial surface of abdomen, intestine, omentum and peritoneum, similar to peritoneal dialysis. Temporary extracorporeal liver support function can be provided for a patient avoiding extracorporeal whole blood perfusion, plasmaphereses or plasmafiltration and associated anticoagulation.

The application of the tubular mass exchange device generally succeeds according to the following principles: (a) temporary surgical implantation of the mass exchange device, in case of additional surgery preferably in the same session; (b) connecting the device with a pump driven bioreactor perfusion system, (c) filling the circuit with perfusate, (d) temporary perfusion of the device and the abdomen in the same circuit.

The concept of using the device involves:
- using the serosa instead of artificial extracorporeal membranes,
- using the heart pump driven microcaoillary vascular abdominal system instead of extracorporeal blood flow via catheters, tubes and pumps,
- avoiding anticoagulation by keeping the blood cells in the vascular endothelial space,
- use of open porous capillaries/tubes in an arterio- venous-like arrangement for fluid distribution for high flow throughput,
- use of a 3-compartment-arrangement for fluid distribution via counter-current flow in two compartments for enhanced mass exchange,
- using albumin in the perfusate for better mass exchange,
- using alternating pressures/fluid levels for further enhancing mass exchange by better reaching all available surfaces, optionally by use of alternating single catheter in-and-out flow techniques as known from single needle dialysis techniques,
- widening the abdominal space by application of all patient fluid substitution during application into the abdominal space,
- further enhancing the fluid level in the abdominal space by additional fluid application in case of good kidney function.

## Claims

1. Tubular body fluid, including abdominal fluid, mass exchange system (2), which is exposable to an environment, wherein the environment defines a first independent compartment for mass exchange in the body, including in the abdomen and thus via the serosa surface with the blood, comprising at least one further second independent compartment, which is composed of a tube arrangement with up to 1000 tubes or capillaries (20), each tube (20) comprising either a microporous tube/capillary wall with pores in the range of 0,01 to 1,0 micrometer or at least one mass exchange aperture (23) with a diameter between 0,05 to 3 mm, wherein the at least one further second independent compartments exhibit each at least one collective main inlet (2a).

2. Tubular system (2) according to claim 1, **characterized in that** the at least one further second independent compartment is composed of 2 to 500 parallel aligned tubes (20).

3. Tubular system (2) according to claim 1, **characterized in that** the at least one further second independent compartment is composed of 2 to 500 meander-like aligned tubes (20).

4. Tubular system (2) according to one of the preceding claims, **characterized in that**
a) two further, a second and a third independent compartment are comprised, which are each composed of a tube arrangement with up to 500 tubes (20),
or
b) three further, a second, a third and a fourth independent compartment are comprised, which are each composed of a tube arrangement with up to 500 tubes (20).

5. Tubular system (2) according to one of the preceding claims, **characterized in that** each independent compartment which is composed of a tube arrangement comprises at least one main inlet and at least one main outlet, preferably at least one collective main inlet and at least one collective main outlet.

6. Tubular system (2) according to one of the claims 1 to 4, **characterized in that** the second compartment comprises at least one main inlet (2a) but no main outlet whereas the third compartment comprises at least one main outlet (2b) but no main inlet, wherein the second and the third compartment are aligned and operable in open perfusion mode.

7. Tubular system (2) according to one of the claims 1 to 6, **characterized in that** the second compartment comprises at least one main inlet (2a) and at least one main outlet whereas the third compartment comprises at least one main outlet (2b) and at least one main inlet, wherein the second and the third compartment are aligned and operable in counter-current cross flow perfusion mode so that mass exchange occurs decentralized between them.

8. Tubular system (2) according to one of the preceding claims, **characterized in that** the tubes or capillaries (20) are composed of medical grade material for temporary implantation, including silicone rubber, polyvinylchloride, polyurethane and polyethersulfone.

9. Tubular system (2) according to one of the preceding claims, **characterized in that** the fluid communicating between first and second or further compartments is enriched with carrier proteins, including albumin, to enhance mass exchange via the serosa membrane.

10. Mass exchange device (100), comprising
a) at least one tubular system (2) according to one of the preceding claims,
b) at least one module (3) for growing and for exploiting the metabolic performance and/or for obtaining micro-organisms (9), in particular for cells, composed of an external housing (30), at least two, preferably three, independent membrane systems (31, 32), at least one of which is designed as hollow-fibre membranes (34) and arranged in the interior (4) of the module (3), these hollow-fibre membranes (34) forming a densely packed spatial reticulate structure (5) and microorganisms (9), which are located in the interstices of the reticulate structure (5) and/or adhere to the hollow-fibre membranes (34), in which the reticulate structure (5) is composed of intersecting and/or superimposed hollow-fibre membranes (34) and is constructed in such a way that the substrate supply and substrate disposal conditions for the microorganisms (9) are virtually identical at any given place in the interior (4) of the module (3), wherein the module (3) features at least one inlet (3a) and at least one outlet (3b),
c) at least one feed line (4a), connecting the at least one outlet (3b) of the at least module (3) with the at least one inlet (2a) of the at least one tubular system (2),
d) at least one feed line (4b), connecting the at least one inlet (3a) of the at least one module (3) with the outlet (2b) of the at least one tubular system (2),
e) at least one pump (4c) being aligned in the at least one feed line (4a) and/or the at least one feed line (4b), and
f) optionally a filter being aligned in the circuit to catch particle aggregates, including protein flakes.

11. Mass exchange device (100) according to the preceding claim, **characterized in that** the densely packed spatial reticulate structure (5) is composed of three independent hollow-fibre membrane systems (31, 32, 33).

12. Mass exchange device (100) according to one of the claims 10 to 11, **characterized in that** an exchangeable flat membrane (11) or capillary membrane (12) is additionally arranged on the external housing (30).

13. Mass exchange device (100) according to one of the claims 10 to 12, **characterized in that** the densely packed reticulate structure (5) is additionally equipped with a further, liquid-impermeable independent capillary system, including for heating or application of physical forces to the cell compartment.

14. Mass exchange device (100) according to one of the claims 10 to 13, **characterized in that** the densely packed reticulate structure (5) is additionally equipped with a further independent capillary system for dialysis purposes.

15. Mass exchange device (100) according to one of the claims 10 to 14, **characterized in that** the external housing (30) is made of a filling composition that pots the capillary ends in dedicated flow head shaped areas of the housing, so that in combination with flow head lids which are fixed, including by gluing or screwing, onto these flow head areas, an access within these flow head areas from the outside to the lumen of the capillaries or of the hollow-fibre membranes is made possible, whereas the at least one inlet (3a) and/or the at least one outlet (3b) is provided with a suitable intake and/or outlet in the flow head (6, 13, 14, 15) which communicates with the respective independent membrane system (31, 32, 33).

16. Mass exchange device (100) according to one of the claims 10 to 15, **characterized in that** the external housing (30) of the module (3) provides one or more accesses (7, 8) which lead into the interior (4), in order to charge the module (3) with micro-organisms (9) and/or to carry out measurements, including pressure-, temperature-and/or pH measurements.

17. Mass exchange device (100) according to the preceding claim, **characterized in that** the accesses (7, 8) continue into the module (3) in the form of perforated or open-ended tubes, allowing the micro-organisms (9) to be distributed uniformly in the interior (4).

18. Use of the tubular system (2) according to one of the claims 1 to 9 and/or the mass exchange device according to one of the claims 10 to 17 as apparatus for medical purposes, especially as apparatus for extracorporeal liver support, for liver detoxification including for albumin dialysis, for assisting the natural liver regeneration, for bridging to liver recovery after liver surgery, for bridging to liver transplant function after initial transplant underfunction, for bridging to donor liver function after small-for size split liver transplantation, for dialysis, for drug, mediator and/or factor admission and/or for drug, mediator and/or factor regulation.
